# EUROPEAN PATENT SPECIFICATION

(11) **EP 2 681 201 B1**
(45) Date of publication and mention of the grant of the patent: **24.08.2016**
(21) Application number: 12712549.0
(22) Date of filing: 05.03.2012
(51) Int. Cl.: C07D 235/26

(54) **A METHOD OF MANUFACTURING 2-ETHOXY-1-(2'-((HYDROXYAMINO)IMINOMETHYL)BIPHENYL-4-YL) METHYL)-1H-BENZO(D)IMIDAZOLE-7-CARBOXYLIC ACID AND ITS ESTERS**
EINE METHODE ZUR HERSTELLUNG VON 2-ETHOXY-1-(2'-((HYDROXYAMINO)IMINOMETHYL)BIPHENYL-4-YL) METHYL)-1H-BENZO(D)IMIDAZOLE-7-CARBONSÄURE UND ESTER DAVON
UNE METHODE DE PRÉPARATION DE L'ACIDE 2-ETHOXY-1-(2'-((HYDROXYAMINO)IMINOMETHYL)BIPHENYL-4-YL) METHYL)-1H-BENZO(D)IMIDAZOLE-7-CARBOXYLIQUE ET SES ESTERS

(30) Priority: 04.03.2011 CZ 20110118
(43) Date of publication of application: 08.01.2014
(73) Proprietor: Zentiva, K.S., 102 37 Praha 10 (CZ)
(72) Inventor: RADL, Stanislav, 250 84 Kvetnice (CZ)
(74) Representative: Jirotkova, Ivana
(86) International application number: PCT/CZ2012/000023
(87) International publication number: WO 2012/119573

(56) References cited:
- EP-A2- 0 520 423
- CN-A- 1 079 966
- CN-A- 102 344 415
- US-A- 5 583 141
- KOHARA Y.; KUBO K.; IMAMIYA E.; WADA T.; INADA Y.; NAKA T.: J MED CHEM., vol. 39, no. 26, 1996, pages 5228-5235, XP002448276, cited in the application
- DATABASE CA [Online] CHEMICAL ABSTRACTS SERVICE, COLUMBUS, OHIO, US; WANG, JIANGUO ET AL: "Improved Synthesis of 5-Oxo-1,2,4-oxadiazole Compounds", XP002676273, retrieved from STN Database accession no. 2005:538981 cited in the application & WANG, JIANGUO ET AL: "Improved Synthesis of 5-Oxo-1,2,4-oxadiazole Compounds", ZHONGGUO YIYAO GONGYE ZAZHI , 35(11), 646-647 CODEN: ZYGZEA; ISSN: 1001-8255, 2004,

## Description

### Technical Field

The invention relates to an improved method of manufacturing 2-ethoxy-1-((2'-((hydroxyamino)iminomethyl)-biphenyl-4-yl)methyl)-1H-benzo[d]imidazole-7-carboxylic acid and its esters (**I**), 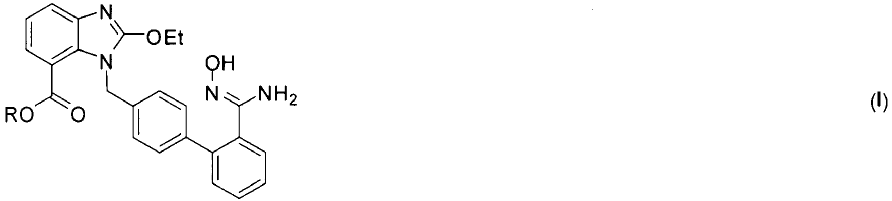 wherein R is either H or an (un)branched C₁-C₄ alkyl, ArCH₂, Ar₂CH, or Ar₃C, wherein Ar is a (un)substituted phenyl, which are suitable intermediates of synthesis of azilsartan (**II**), a potent antagonist of angiotensin II in AT1 receptors, which is used to treat hypertension in the form of the prodrug azilsartan medoxomil (**III**).

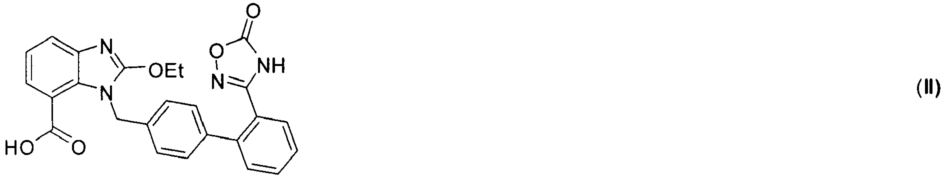

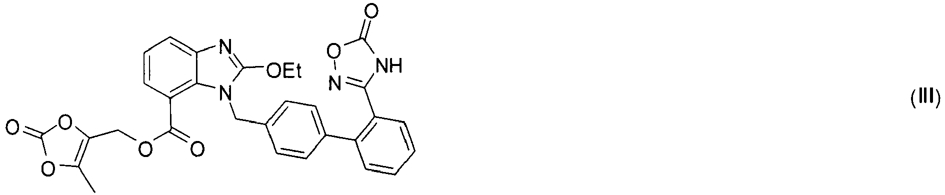

### Background Art

Azilsartan (**II**) is manufactured, according to published patents (US patent 5,583,141 and EP 0,520,423), from the starting methyl 1-((2'-cyanobiphenyl-4-yl)methyl)-2-ethoxy-1*H-*benzo[*d*]imidazole-7-carboxylate (**IVa**). This compound, which is a well-known intermediate of synthesis of candesartan, is first transformed, by a reaction with hydroxylamine generated *in situ* from hydroxylamine hydrochloride, to methyl 2-ethoxy-1-((2'-((hydroxyamino)-iminomethyl)biphenyl-4-yl)methyl)-1*H*-benzo[*d*]imidazole-7-carboxylate (**Ia**), which is then converted to methyl 2-ethoxy-1-((2'-(5-oxo-4,5-dihydro-1,2,4-oxadiazol-3-yl)biphenyl-4-yl)methyl)-1*H*-benzo[*d*]imidazole-7-carboxylate (**Va**), from which azilsartan (**II**) is obtained by saponification.

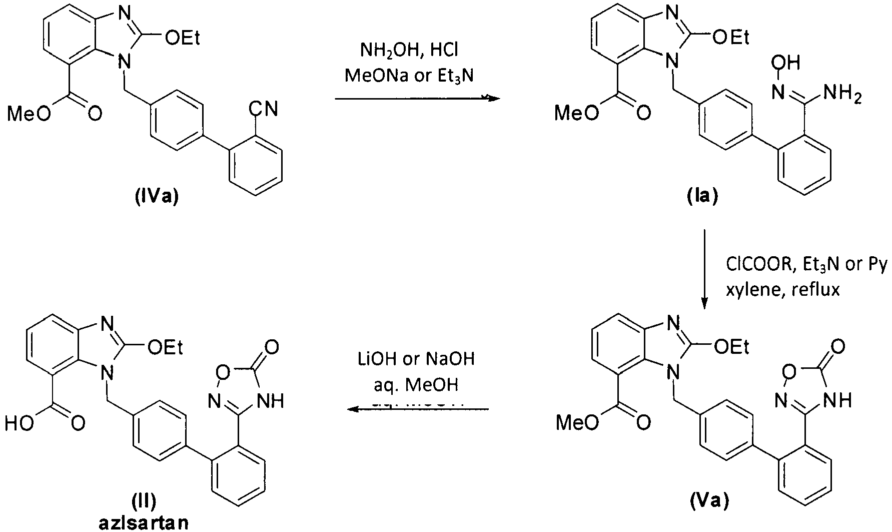

In the above mentioned patents the transformation of the nitrile (**IVa**) to the amidoxime (**Ia**) is achieved by four-hour heating with hydroxylamine, generated *in situ* by a reaction of hydroxylamine hydrochloride, with sodium methanolate in DMSO at the temperature of 90 °C, the reported yield of the compound (**Ia**) being 90%. In more recent publications the same authors (Kohara Y., Imamiya E., Kubo K., Wada T., Inada Y., Naka T.: Bioorg. Med. Chem. Lett. 1995, 5(7), 1903-1908; Kohara Y., Kubo K., Imamiya E., Wada T., Inada Y., Naka T.: J. Med. Chem. 1996, 39(26), 5228-5235) have generated hydroxylamine by means of triethylamine and carried out the reaction at the temperature of 75 °C. The reported reaction time is 15 hours this time; after dilution of the reaction mixture with water the crude product is obtained by extraction with ethyl acetate in the form of an organic solution, which is further washed with diluted hydrochloric acid and the resulting aqueous solution of amidoxime (**Ia**) hydrochloride is alkalized and extracted back with ethyl acetate, to afford, after processing, the amidoxime (**Ia**) in the 55% yield.

### Disclosure of Invention

The invention provides a new efficient method for preparing 2-ethoxy-1-((2'-((hydroxyamino)iminomethyl)biphenyl-4-yl)methyl)-1*H*-benzo[*d*]imidazole-7-carboxylic acid and its esters (**I**). These compounds are important intermediates in the synthesis of azilsartan.

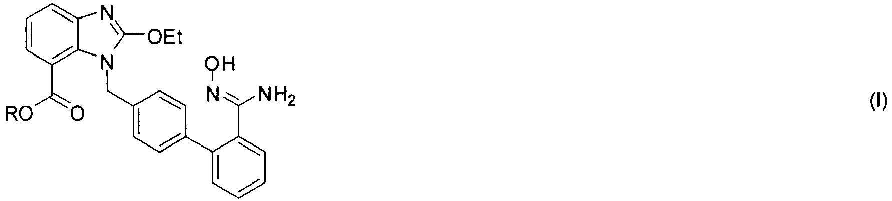

The method consists in a reaction of the compounds of formula (**IV**) with an aqueous solution of hydroxylamine.

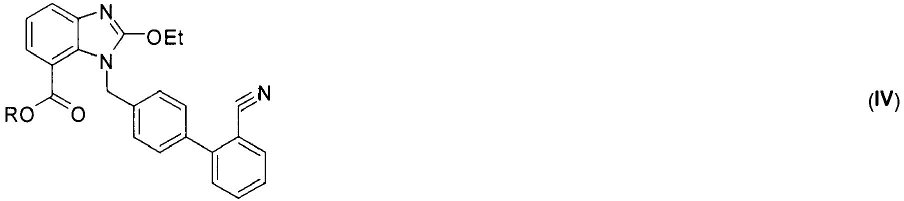

The resulting compounds of formula (**I**) are produced in a high yield and this method of preparation reduces formation of undesired products.

### Detailed description of the invention

The invention relates to a new efficient method of preparation of 2-ethoxy-1-((2'-((hydroxyamino)iminomethyl)biphenyl-4-yl)methyl)-1*H*-benzo[*d*]imidazole-7-carboxylic acid and its esters (I), wherein R is either H or an (un)branched C₁-C₄ alkyl, ArCH₂, Ar₂CH, or Ar₃C, wherein Ar is a (un)substituted phenyl. These compounds are important intermediates in the synthesis of azilsartan.

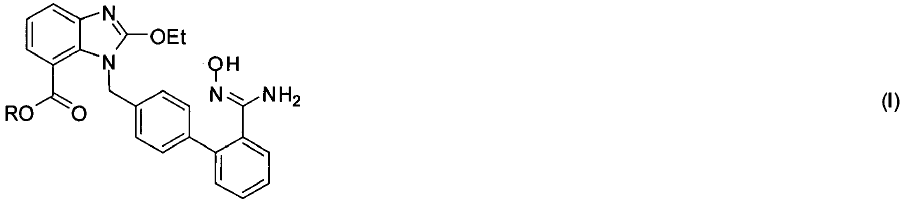

The method consists in a reaction of the compounds of formula (**IV**) 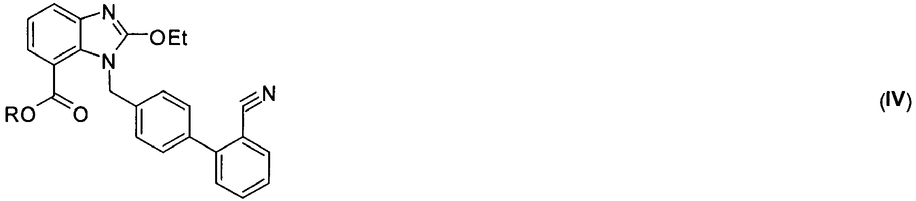 with an aqueous solution containing 20 to 80% by weight of hydroxylamine in a polar aprotic solvent, or in a mixture of polar aprotic solvents. The resulting compounds of formula (I) are produced in a high yield and this method of preparation suppresses formation of undesired products, especially formation of compounds of formula (**VI**), 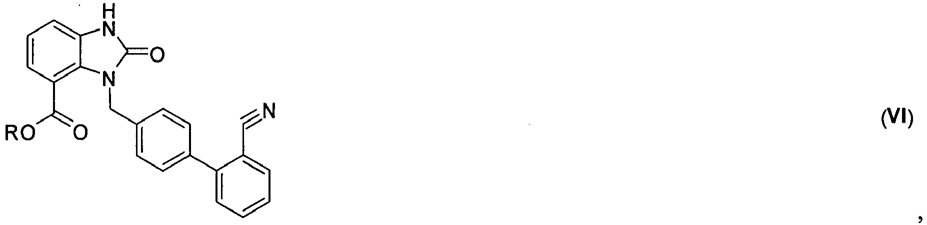 wherein R is as defined above.

For the reaction, an aqueous solution of hydroxylamine is used at a concentration of 20 to 80% by weight, in a preferable embodiment, a solution with a concentration of 40-60% by weight. The polar aprotic solvents to be used include dimethyl sulfoxide (DMSO), *N*,*N*-dimethyl formamide (DMF), *N*,*N*-dimethyl acetamide (DMAc), 1-methylpyrrolidone (NMP), 1,1,3,3-tetramethylurea (TMU), 1,3-dimethylimidazolidin-2-one (DMI), 1,3-dimethyl-3,4,5,6-tetrahydro-2(1*H*)-pyrimidinone (DMPU), hexamethylphosphoramide (HMPA), or mixtures thereof, or possibly mixtures with other solvents. In a preferable embodiment, DMSO or NMP is used. The reaction is carried out at a temperature in the range of 40 to 100°C, preferably at a temperature in the range of 50-90°C.

The reaction mixture is heated for a sufficiently long time in the range of 12 to 48 hours; after the reaction is complete, the reaction mixture is processed by diluting with water or by pouring of the reaction mixture into water. The separated product is then isolated by filtration or centrifugation.

The crude compounds of formula (**I**) are obtained in the purity of at least 95% with a low content of the compounds (**VI**). If necessary, the crude compounds of formula (**I**) can be purified by crystallization from suitable solvents, including esters of aliphatic acids (e.g., methyl acetate, ethyl acetate, isopropyl acetate), amides (*N*,*N*-dimethyl formamide, *N,N-*dimethyl acetamide, 1-methylpyrrolidone), ethers (e.g., tetrahydrofuran, dioxan, 1,2-dimethoxyethane, 1-methoxy-2-(2-methoxyethoxy)ethane, or mixture of these solvents.

It has been found in repeating the prior described processes of synthesis of amidoxime (**Ia**) that the starting compound, as well as a considerable amount of desethyl nitrile (**VIa**), is present in the reaction mixture besides the desired product. The side product (**VIa**) is formally a product of hydrolysis of the ethoxy group in position 2 of the benzimidazole skeleton of the nitrile (**IVa**). 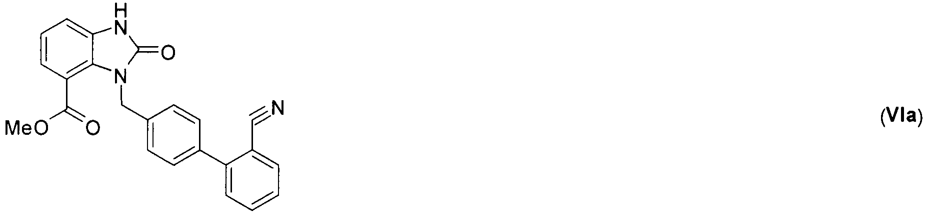 wherein Me = CH₃

By a reproduction of the method according to EP 0,520,423 (see Examples 1 and 5), crude products containing 17.6% and 19.7% , resp. of compound (**VIa**) have been prepared. Repeating of the experiments in a strictly anhydrous environment provided comparable results. Formation of the corresponding desethyl nitriles was also detected in the same reactions of the ethyl ester (**IVb**) and benzyl ester (**IVc**) (see Examples 6, 8 and 9). It has been found by modifying the above-mentioned methods that neither a temperature change nor a change of the base results in a substantial reduction of the amount of this side product (see Examples 3, 6, 7 and 9).

Although the original patents or the cited publications do not mention formation of the compound (**VIa**) at all, their authors apparently had faced problems with such formation, which they solved by the above mentioned purification of the crude product, which is actually able to remove this side product from the crude product (see Examples 2 and 7). By repeating the method according to EP 0,520,423, followed by crystallization, we have found out that a significant amount of the compound (**VIa**) can be reduced; e.g., from 18.9% to 1.3%. However, such operations result in considerable reduction of the yield

The present invention is based on the finding that under certain strictly defined conditions, nearly complete suppression of the formation of desethyl nitriles (**VI**) can be achieved. It has been found out that formation of these compounds in the reaction mixture during *in situ* generation of the hydroxylamine base occurs both in the presence of an excess of a hydroxylamine salt (e.g. hydroxylamine hydrochloride) and in the presence of an excess of most bases commonly used to release the hydroxylamine base. In screening the conditions we have found out that this problem can be solved either by using weak bases to release hydroxylamine and by performing the subsequent addition of hydroxylamine in some solvents at an only slightly elevated temperature. A typical example is the use of NaHCO₃ or NaOAc in boiling methanol or in a toluene-methanol mixture. Unfortunately, under these conditions the reaction is very slow and several days' heating is necessary to achieve a complete conversion. With regard to the structure of the side products (**IV**) it is very surprising to find out that if the reaction is carried out using an aqueous solution of hydroxylamine, desethyl nitriles are virtually not generated at all.

In extensive screening of various modifications of the above mentioned methods, both hydroxylamine hydrochloride and hydroxylamine sulphate, and hydroxylamine phosphate were used as sources of hydroxylamine. For the release of the hydroxylamine base, a number of species have been tested, including amines (e.g., triethylamine, DIPEA, DBU, 1,8-bis-(dimethylamino)naphthalene), alcoholates (e.g., MeONa, MeOK, EtONa, t-BuONa, t-BuOK, t-AmONa, t-AmOK), hydroxides (NaOH, KOH, CsOH), carbonates (Na₂CO₃, K₂CO₃, CsCO₃), hydrogen carbonates (NaHCO₃, KHCO₃) and acetates (NaOAc, KOAc), in a number of solvents and their mixtures. A considerable reduction of formation of desethyl nitriles (**VI**) was achieved when the reaction was carried out in methanol or its mixture with toluene when using weak bases. The use of NaHCO₃ or NaOAc in methanol or its mixture with toluene under reflux has proved to be the most convenient. Unfortunately, under the given conditions the reaction is considerably slow and a significant portion of the starting compound remains unreacted even after 48 hours.

A considerable improvement was achieved by using an aqueous solution of hydroxylamine. This method does not require addition of any inorganic or organic bases and therefore there is no risk of contamination of the product. The make-up of the reaction is very simple and consists in mere heating of the starting nitrile (**IV**) with aqueous hydroxylamine in a suitable solvent. After achievement of the desired conversion water is added (or the reaction mixture is poured into water), the resulting product (**I**) is separated in the form of a well filterable precipitate and, after washing, a highly pure product is obtained. It can be further recrystallized, or preferably used, after drying, directly for further reaction.

Aqueous hydroxylamine containing 20 - 80 weight percent of hydroxylamine, preferably 40 - 60%, can be used for the reaction of nitriles (**IV**). The use of aqueous hydroxylamine containing 50 weight % of hydroxylamine is the most convenient from the point of view of commercial availability.

The reaction runs even at the laboratory temperature, but with regard to its duration it is more advantageous to perform it at an elevated temperature, preferably in the range of 40 to 100 °C. Even at higher temperatures no occurrence of a more significant amount of desethyl nitriles (**VI**) has been observed. It is most preferable to carry out the reaction at a temperature of 50 - 90 °C.

Due to limited solubility of the starting nitriles (**IV**) the number of suitable solvents is limited to polar aprotic solvents, such as sulfoxides (e.g. DMSO), amides (e.g. *N,N-*dimethyl formamide (DMF), *N*,*N*-dimethyl acetamide (DMAc), 1-methylpyrrolidone (NMP)), urea derivatives (e.g. 1,1,3,3-tetramethylurea (TMU), 1,3-dimethylimidazolidin-2-one (DMI), 1,3-dimethyl-3,4,5,6-tetrahydro-2(1*H*)-pyrimidinone (DMPU)), hexamethylphosphoramide (HMPA), or their mixtures. The reaction can also be performed in a mixture of the above mentioned solvents with other co-solvents such as esters (e.g. alkyl acetates such as methyl acetate or ethyl acetate) or aromatic hydrocarbons (e.g. toluene), or diglyme.

During the development of the synthesis of alkyl 2-ethoxy-1-((2'-((hydroxyamino)-iminomethyl)biphenyl-4-yl)methyl)-1*H*-benzo[*d*]imidazole-7-carboxylates, the reactions were routinely monitored by means of HPLC in a HP 1050 device equipped with a Phenomenex Luna 5µ C18(2) column, 250 x 4.6 mm, equipped with a 227nm UV detector. Phase A: 1.2 g of NaH₂PO₄/l 1 of water (pH = 3.0; Phase B: methanol.

The invention is elucidated in more detail in the following working examples. The examples, which illustrate the improvement of the method according to the invention, only have an illustrative character and do not limit the scope of the invention in any respect.

### Working Examples

### Comparative examples:

### Example 1

### Methyl 2-ethoxy-1-((2'-((hydroxyamino)iminomethyl)biphenyl-4-yl)methyl)-1H-benzo[d]-imidazole-7-carboxylate (Ia) - reproduction of the process (US 5,583,141; EP 0,520,423)

A 30% solution of MeONa in MeOH (9.1 g) was added to a stirred mixture of finely divided hydroxylamine hydrochloride (3.5 g, 50 mmol) in dry DMSO (40 ml) and the mixture was stirred under nitrogen at the laboratory temperature for 10 minutes. Then, the nitrile (**IVa;** 4.11 g, 10 mmol) was added and the mixture was stirred at the temperature of 90 °C for 4 hours. According to HPLC the mixture contains 32.3% of the product (**Ia**), 49.9 % of the starting nitrile (**IVa**) and 17.6 % of the desethyl nitrile (**VIa**). The reaction mixture was cooled to the laboratory temperature, then water (25 ml) was added, the mixture, heated by the dilution, was cooled and stirred at the laboratory temperature for 30 minutes. The insoluble fraction was aspirated, washed with water and air-dried. 3.9 g of a substance were obtained, containing, according to HPLC, 30.7 % of the product (**Ia**), 49.8% of the starting nitrile (**IVa**) and 18.9 % of the desethyl nitrile (**VIa**).

### Example 2

### Methyl 2-ethoxy-1-((2'-((hydroxyamino)iminomethyl)biphenyl-4-yl)methyl)-1H-benzo[d]-imidazole-7-carboxylate (Ia) - modification of the process (US 5,583,141; EP 0,520,423)

The product obtained using the method of Example 1 (3.9 g) was dissolved in dichloromethane (400 ml) and the resulting, still turbid solution was washed with 4 x 50 ml of 5 % HCl. Thus obtained aqueous solution was alkalinized with a concentrated (20 %) solution of sodium hydroxide and the resulting precipitate was aspirated and washed with water. After drying 1.6 g (the total yield of Example 1 and Example 2 is 36.0 %) of the substance (**Ia**) were obtained, containing, according to HPLC, 96.7 % of the product (**Ia**), an undetectable amount of the starting nitrile (**IVa**) and 1.3 % of the desethyl nitrile (**VIa**).

### Example 3

### Methyl 2-ethoxy-1-((2'-((hydroxyamino)iminomethyl)biphenyl-4-yl)methyl)-1H-benzo[d]-imidazole-7-carboxylate (Ia) - modification of the process (US 5,583,141; EP 0,520,423)

Using the method described in Example 1 but carrying the reaction out at the temperature of 90 °C for 20 hours a reaction mixture was obtained, containing, according to HPLC 44.7 % of the product (**Ia**), 11.4 % of the starting nitrile (**IVa**) and 40.5 % of the desethyl nitrile (**VIa**).

### Example 4

### Methyl 2-ethoxy-1-((2'-((hydroxyamino)iminomethyl)biphenyl-4-yl)methyl)-1H-benzo[d]-imidazole-7-carboxylate (Ia) - modification of the process (US 5,583,141; EP 0,520,423)

Using the method described in Example 1 but carrying the reaction out at the temperature of 75 °C for 24 hours a reaction mixture was obtained, containing, according to HPLC, 30.7 % of the product (**Ia**), 49.8 % of the starting nitrile (**IVa**) and 18.8 % of the desethyl nitrile (**VIa**).

### Example 5

### Methyl 2-ethoxy-1-((2'-((hydroxyamino)iminomethyl)biphenyl-4-yl)methyl)-1H-benzo[d]-imidazole-7-carboxylate (Ia) - reproduction of the process (J. Med. Chem. 1996, 39(26), 5228-5235)

Triethylamine (1.7 ml = 1.24 g) was added to a suspension of hydroxylamine hydrochloride (0.85 g, 12.2 mmol) in DMSO (4 ml) and the mixture was stirred at the laboratory temperature for 15 minutes. Then the thick slurry was aspirated and washed with tetrahydrofuran (10 ml). The filtrate was concentrated in vacuo (most of tetrahydrofuran was evaporated). The nitrile (**IVa;** 1.03 g, 2.5 mmol) was added to this solution and the mixture was stirred at the temperature of 75 °C for 15 hours. According to HPLC, the mixture contains 44.2 % of the product (**Ia**), 35.2 % of the starting nitrile (**IVa**) and 19.7 % of the desethyl nitrile (**VIa**).

### Example 6

### Ethyl 2-ethoxy-1-((2'-((hydroxyamino)iminomethyl)biphenyl-4-yl)methyl)-1H-benzo[d]-imidazole-7-carboxylate (Ib) -modification of the process (US 5,583,141; EP 0,520,423)

Using the method described in Example 1 but carrying the reaction out with the ethyl ester (IVb; 8.5 g, 20 mmol) at the temperature of 90 °C for 14 hours, a reaction mixture was obtained containing, according to HPLC, 68.8 % of the product (**Ib**), 5.2 % of the starting nitrile (IVb) and 22.9 % of the desethyl nitrile (**VIb**). After pouring the reaction mixture onto water the resulting precipitate was aspirated, washed with water and dried (9.45 g). A solution of this substance in dichloromethane (900 ml) was shaken with 5 % HCl (4 x 100 ml), the combined aqueous fractions were alkalized with concentrated NaOH and the resulting fine suspension was stirred at the laboratory temperature overnight. Then the insoluble fraction was aspirated, washed with water and dried. After crystallization from ethyl acetate 4.7 g (51.2 %) of creamy crystals were obtained, containing, according to HPLC, 96.8 % of the product (**Ib**), 0 % of the starting nitrile (**IVb**) and 0.2 % of the desethyl nitrile (**VIb**).

### Example 7

### Ethyl 2-ethoxy-1-((2'-((hydroxyamino)iminomethyl)biphenyl-4-yl)methyl)-1H-benzo[d]-imidazole-7-carboxylate (Ib) - modification of the process (US 5,583,141; EP 0,520,423)

Using the method described in Example 4 but carrying the reaction out with the ethyl ester (**IVb**; 1.7 g, 4 mmol) at the temperature of 75 °C for 24 hours, a reaction mixture was obtained containing, according to HPLC, 58.9 % of the product (**Ib**), 8.3 % of the starting nitrile (**IVb**) and 20.7 % of the desethyl nitrile (**VIb**). After pouring the reaction mixture onto water the resulting precipitate was aspirated, washed with water and dried (1.7 g). A solution of this substance in dichloromethane (300 ml) was shaken with 5 % HCl (3 x 35 ml), the combined aqueous fractions were alkalized with concentrated NaOH and the resulting fine suspension was extracted with dichloromethane. After washing with water the extract was dried with MgSO₄ and, after evaporation, 0.8 g (43.6 %) of the evaporation product was obtained (100 % pure product) according to HPLC.

### Example 8

### Ethyl 2-ethoxy-1-((2'-((hydroxyamino)iminomethyl)biphenyl-4-yl)methyl)-1H-benzo[d]-imidazole-7-carboxylate (Ib) - modification of the process (J. Med. Chem. 1996, 39(26), 5228-5235)

Using the method described in Example 5 but carrying the reaction out with the ethyl ester (**IVb**), a reaction mixture was obtained containing, according to HPLC, 42.5 % of the product (**Ib**), 39.7 % of the starting nitrile (**IVb**) and 17.6 % of the desethyl nitrile (**VIb**).

### Example 9

### Ethyl 2-ethoxy-1-((2'-((hydroxyamino)iminomethyl)biphenyl-4-yl)methyl)-1H-benzo[d]-imidazole-7-carboxylate (Ib) - modification of the process (J. Med. Chem. 1996, 39(26), 5228-5235)

Triethylamine (3.4 ml = 2.48 g) was added to a suspension of hydroxylamine hydrochloride (1.7 g, 25 mmol) in DMSO (8 ml) and the mixture was stirred at the laboratory temperature for 15 minutes. The thick slurry was then aspirated and washed with tetrahydrofuran (10 ml). The filtrate was concentrated in vacuo (most tetrahydrofuran evaporated). The nitrile (**IVa**; 2.06 g, 5 mmol) was added to this solution and the mixture was stirred at the laboratory temperature for 15 minutes. Then, the mixture was partitioned into 4 reaction vials and these were stirred at the laboratory temperature (A), at the temperature of 50 °C (B), 75 °C (C) and 100 °C (D) for 48 hours. Then the reaction mixtures were analyzed using HPLC. The results are summarized in Table I.

**Table I - Composition of the reaction mixture of Example 9**

| Entry | Temperature | % **Ia** | % **IVa** | % **VIa** | % Others |
|---|---|---|---|---|---|
| A | 20 °C | 3.2 | 96.1 | 0.4 | 0.3 |
| B | 50 °C | 33.1 | 49.6 | 11.7 | 5.6 |
| C | 75 °C | 54.4 | 1.0 | 32.8 | 11.8 |
| D | 100 °C | 29.4 | 0 | 27.0 | 43.6 |

### Example 10

### Benzyl 2-ethoxy-1-((2'-((hydroxyamino)iminomethyl)biphenyl-4-yl)methyl)-1H-benzo[d]-imidazole-7-carboxylate (Ic) - modification of the process (US 5,583,141; EP 0,520,423)

Using the method described in Example 4 but carrying the reaction out with the benzyl ester (**IVc**) at 75 °C for 24 hours, a reaction mixture was obtained containing, according to HPLC, 52.8 % of the product (**Ic**), 13.4 % of the starting nitrile (**IVc**) and 23.7 % of the desethyl nitrile (**VIc**).

### Example 11

### Ethyl 2-ethoxy-1-((2'-((hydroxyamino)iminomethyl)biphenyl-4-yl)methyl)-1H-benzo[d]-imidazole-7-carboxylate (Ib) - using NaHCO₃ as the base

Sodium hydrogen carbonate (0.2 g, 2.4 mmol) was added to a suspension of hydroxylamine hydrochloride (0.15 g, 2.2 mmol) in methanol (10 ml) and the mixture was stirred at the laboratory temperature for 10 minutes. Then, the nitrile (**IVb**, 0.43 g, 1 mmol) was added and the mixture was heated under moderate reflux for 24 h. According to HPLC, the mixture contains 32.1 % of the product (**Ib**), 65.9 % of the starting nitrile (**IVb**) and 2.0 % of the desethyl nitrile (**VIb**). Another portion of sodium hydrogen carbonate (0.2 g, 2.4 mmol) and hydroxylamine hydrochloride (0.15 g, 2.2 mmol) was added to the mixture and the mixture was heated under moderate reflux for another 24 h. According to HPLC, the mixture contains 56.2% of the product (**Ib**), 33.2 % of the starting nitrile (**IVb**) and 5.1 % of the desethyl nitrile (**VIb**). After cooling the mixture was poured into water (50 ml), the insoluble fraction was aspirated and washed with water. After drying, 0.35 g of a substance was obtained, containing, according to HPLC, 54.7 % of the product (**Ib**), 31.3 % of the starting nitrile (**IVb**) and 6.8 % of the desethyl nitrile (**VIb**).

### Example 12

### Methyl 2-ethoxy-1-((2'-((hydroxyamino)iminomethyl)biphenyl-4-yl)methyl)-1H-benzo[d]-imidazole-7-carboxylate (Ia) - using NaOAc as the base

Sodium acetate (0.2 g, 2.4 mmol) was added to a suspension of hydroxylamine hydrochloride (0.15 g, 2.2 mmol) in toluene (10 ml) and the mixture was stirred at the laboratory temperature for 10 minutes. Then, the nitrile (**IVa**, 0.41 g, 1 mmol) was added and the mixture was heated under moderate reflux for 24 h. According to HPLC, the mixture contains 36.2 % of the product (**Ia**), 58.7 % of the starting nitrile (**IVa**) and 3.4 % of the desethyl nitrile (**VIa**). Another portion of sodium acetate (0.2 g, 2.4 mmol) and hydroxylamine hydrochloride (0.15 g, 2.2 mmol) was added to the mixture and the mixture was heated under moderate reflux for another 24 h. According to HPLC, the mixture contains 58.1 % of the product (**Ia**), 32.5 % of the starting nitrile (**IVa**) and 7.1 % of the desethyl nitrile (**VIa**). The reaction mixture was poured into water (50 ml) under stirring, the insoluble fraction was aspirated and, after drying, 0.26 g of a substance was obtained containing, according to HPLC, 78.3 % of the product (**Ia**), 3.6 % of the starting nitrile (**IVa**) and 13.2 % of the desethyl nitrile (**VIa**). The filtrate was partitioned and the toluene layer was analyzed with HPLC - it contains 10.6 % of the product (**Ia**), 85.9 % of the starting nitrile (**IVa**) and 2.7 % of the desethyl nitrile (**VIa**).

### Working Examples using aqueous hydroxylamine:

### Example 13

### Methyl 2-ethoxy-1-((2'-((hydroxyamino)iminomethyl)biphenyl-4-yl)methyl)-1H-benzo[d]-imidazole-7-carboxylate (Ia) - using aqueous hydroxylamine

50 % aqueous hydroxylamine (0.3 ml) was added to a solution of the nitrile (**IVa**; 0.41 g, 1 mmol) in the corresponding solvent (4 ml) and the mixture was stirred in a reaction vial at the temperature of 75 °C for 24 hours and the reaction mixtures were analyzed with HPLC. The results are summarized in Table II.

**Table II - Composition of the reaction mixture of Example 12**

| Entry | Solvent | % **Ia** | % **IVa** | % **VIa** | % Others |
|---|---|---|---|---|---|
| A | DMSO | 84.3 | 7.6 | 2.2 | 5.9 |
| B | DMF | 61.9 | 16.1 | 13.7 | 8.3 |
| C | DMAc | 45.3 | 37.1 | 15.0 | 2.5 |
| D* | NMP | 76.2 | 15.1 | 0.6 | 8.1 |
| E | DMI | 81.5 | 12.1 | 2.1 | 4.3 |
| F | DMPU | 86.1 | 3.6 | 1.2 | 9.1 |

| | | | | | |
|---|---|---|---|---|---|
| - After another 24 hours the mixture contained, according to HPLC, 81.6 % of the product (**Ia**), 2.7 % of the starting nitrile (**IVa**) and 1.2 % of the desethyl nitrile (**VIa**). | | | | | |

### Example 14

### Ethyl 2-ethoxy-1-((2'-((hydroxyamino)iminomethyl)biphenyl-4-yl)methyl)-1H-benzo[d] imidazole-7-carboxylate (Ib) - using aqueous hydroxylamine

50 % aqueous hydroxylamine (0.3 ml) was added to a solution of the nitrile (**IVb**; 0.43 g, 1 mmol) in the corresponding solvent (4 ml) and the mixture was stirred in a reaction vial at the temperature of 75 °C for 24 hours and the reaction mixtures were analyzed with HPLC. The results are summarized in Table III.

**Table III - Composition of the reaction mixture of Example 13**

| Entry | Solvent | % **Ib** | % **IVb** | % **VIb** | % Others |
|---|---|---|---|---|---|
| A | DMSO | 88.0 | 7.1 | 1.4 | 3.5 |
| B | DMF | 71.2 | 16.5 | 5.8 | 6.5 |
| C | DMAc | 43.7 | 41.3 | 12.5 | 2.5 |
| D | NMP | 76.7 | 17.6 | 1.0 | 4.7 |
| E | DMI | 81.2 | 15.4 | 1.5 | 1.9 |
| F | DMPU | 87.8 | 5.3 | 0.9 | 6.0 |

### Example 15

### Ethyl 2-ethoxy-1-((2'-((hydroxyamino)iminomethyl)biphenyl-4-yl)methyl)-1H-benzo[d]-imidazole-7-carboxylate (Ib) - using aqueous hydroxylamine

25% aqueous hydroxylamine (1.5 ml) was added to a solution of the nitrile (**IVb**; 0.86 g, 2 mmol) in DMSO (8 ml) and the mixture was stirred at the temperature of 75 °C for 24 hours. The cooled reaction mixture was poured onto water (25 ml), the insoluble fraction was aspirated, washed with water and dried. g of a product was obtained containing, according to HPLC, 89.6 % of the product (**Ib**), 6.3 % of the starting nitrile (**IVb**) and 2.5 % of the desethyl nitrile (**VIb**).

### Example 16

### Ethyl 2-ethoxy-1-((2'-((hydroxyamino)iminomethyl)biphenyl-4-yl)methyl)-1H-benzo[d]-imidazole-7-carboxylate (Ib) - using aqueous hydroxylamine

A 50 % (w/w) aqueous solution of hydroxylamine (3 ml) was added to a stirred mixture of the nitrile (**IVb**, 8.6 g, 20 mmol) and DMSO (60 ml) and the mixture was stirred under nitrogen at the temperature of 75 °C for 17 h. Then, another 50 % aqueous hydroxylamine (2 ml) was added and heating was continued for another 7 h. According to HPLC, the mixture contains 72.8 % of the product (**Ib**), 20.2 % of the starting nitrile (**IVb**) and 2.3 % of the desethyl nitrile (**VIb**). After addition of another 50 % aqueous hydroxylamine (2 ml) the heating was continued for another 12 h. According to HPLC, the mixture contains 91.3 % of the product (**Ib**), 0.5 % of the starting nitrile (**IVb**) and 2.9 % of the desethyl nitrile (**VIb**). After cooling, water (50 ml) was added dropwise, the mixture was stirred for 30 minutes and then the insoluble fraction was aspirated and washed with water (100 ml). After drying, 8.9 g of the product (97.1 %) were obtained containing, according to HPLC, 98.6 % of the product (**Ib**), 0.3 % of the starting nitrile (**IVb**) and 0.2 % of the desethyl nitrile (**VIb**). By crystallizing a sample from ethyl acetate, an absolutely pure product (according to HPLC) was obtained, having a melting point of 209-211 °C. ¹H NMR spectrum (DMSO-*d6*): ¹H NMR (500 MHz, DMSO) δ (ppm): 9.19 (bs, 1H, OH), 7.69 (dd, *J* = 7.9, 1.2 Hz, 1H, Ar), 7.46 (dd, *J* = 7.9, 1.2 Hz, 1H, Ar), 7.45-7.32 (m, 3H, Ar), 7.35 (d, *J* = 8.4 Hz, 2H, Ar), 7.27 (dd, *J* = 7.6, 1.5 Hz, 1H, Ar), 7.19 (t, *J* = 7.9 Hz, 1H, Ar), 6.93 (d, *J* = 8.4 Hz, 2H, Ar), 5.55 (bs, 2H, NH₂), 5.53 (s, 2H, N-CH₂-Ar), 4.62 (q, *J* = 7.1 Hz, 2H, OCH₂CH₃), 4.22 (q, *J* = 7.1 Hz, 2H, COOCH₂CH₃), 1.42 (t, *J* = 7.1 Hz, 3H, OCH₂CH₃), 1.18 (t, *J* = 7.1 Hz, 3H, COOCH₂CH₃).

### Example 17

### Methyl 2-ethoxy-1-((2'-((hydroxyamino)iminomethyl)biphenyl-4-yl)methyl)-1H-benzo[d]-imidazole-7-carboxylate (Ia) - using aqueous hydroxylamine

Following the procedure described in Example 16 using the nitrile (**IVa**), 96.3 % of the product were obtained containing, according to HPLC, 96.9 % of the product (**Ia**), 1.6% of the starting nitrile (**IVa**) and 0.7 % of the desethyl nitrile (**VIa**). By crystallizing a sample from methyl acetate, an absolutely pure product (according to HPLC) was obtained, having a melting point of 208-210 °C. ¹H NMR spectrum (DMSO-*d6*): ¹H NMR (500 MHz, DMSO) δ (ppm): 9.18 (s, 1H, OH), 7.70 (dd, *J* = 7.9, 1.2 Hz, 1H, Ar), 7.46 (dd, *J* = 7.9, 1.2 Hz, 1H, Ar), 7.45-7.33 (m, 3H, Ar), 7.35 (d, *J* = 8.3 Hz, 2H, Ar), 7.29 (dd, *J* = 7.6, 1.5 Hz, 1H, Ar), 7.19 (t, *J* = 7.9 Hz, 1H, Ar), 6.94 (d, *J* = 8.3 Hz, 2H, Ar), 5.55 (bs, 2H, NH₂), 5.51 (s, 2H, N-CH₂-Ar), 4.62 (q, *J* = 7.1 Hz, 2H, OCH₂CH₃), 3.71 (s, 3H, OCH₃), 1.42 (t, *J* = 7.1 Hz, 3H, OCH₂CH₃)

### Example 18

### Benzyl 2-ethoxy-1-((2'-((hydroxyamino)iminomethyl)biphenyl-4-yl)methyl)-1H-benzo[d]-imidazole-7-carboxylate (Ic) - using aqueous hydroxylamine

Following the procedure described in Example 16 using the nitrile (**IVc**), 89.7 % of the product were obtained containing, according to HPLC, 87.7 % of the product (**Ic**), 2.7 % of the starting nitrile (**IVc**) and 3.5 % of the desethyl nitrile (**VIc**). By crystallizing a sample from isopropyl acetate, an absolutely pure product (according to HPLC) was obtained, having a melting point of 171-173°C. ¹H NMR spectrum (DMSO-*d6*): 9.19 (s, 1H, OH), 7.70 (dd, *J* = 7,9, 1.2 Hz, 1H, Ar), 7.51 (dd, *J* = 7.9, 1.2 Hz, 1H, Ar), 7.45-7.29 (m, 8H, Ar), 7.31 (d, *J* = 8.4 Hz, 2H, Ar), 7.23 (dd, *J* = 7.6, 1.5 Hz, 1H, Ar), 7.19 (t, *J* = 7.9 Hz, 1H, Ar), 6.90 (d, *J* = 8.4 Hz, 2H, Ar), 5.54 (bs, 2H, NH₂), 5.53 (s, 2H, N-CH₂-Ar), 5.27 (s, 2H, COOCH₂-Ph), 4.63 (q, *J* = 7.1 Hz, 2H, OCH₂CH₃), 1.43 (t, *J* = 7.1 Hz, 3H, OCH₂CH₃).

### Example 19

### Benzhydryl 2-ethoxy-1-((2'-((hydroxyamino)iminomethyl)biphenyl-4-yl)methyl)-1H-benzo-[d]imidazole-7-carboxylate (Id) - using aqueous hydroxylamine

Following the procedure described in Example 16 using the nitrile (**IVd**), 84.9 % of the product were obtained containing, according to HPLC, 93.7 % of the product (**Id**), 4.2 % of the starting nitrile (**IVd**) and 2.1 % of the desethyl nitrile (**VId**). By crystallizing a sample from isopropyl acetate, an absolutely pure product (according to HPLC) was obtained, having a melting point of 234 - 237 °C. ¹H NMR spectrum (DMSO-*d6*): 9.19 (s, 1H, OH), 7.76-7.70 (m, 2H, Ar), 7.53-7.44 (m, 4H, Ar), 7.42-7.28 (m, 9H, Ar), 7.25 (t, *J* = 7.9 Hz, 1H, Ar), 7.17 (d, *J* = 8.3 Hz, 2H, Ar), 7.10-6.99 (m, 2H, Ar), 6.82 (d, *J* = 8.3 Hz, 2H, Ar), 5.53 (bs, 2H, NH₂), 5.48 (s, 2H, N-CH₂-Ar), 4.63 (q, *J* = 7.1 Hz, 2H, OCH₂CH₃), 1.43 (t, *J* = 7.1 Hz, 3H, OCH₂CH₃);

### Example 20

### Trityl 2-ethoxy-1-((2'-((hydroxyamino)iminomethyl)biphenyl-4-yl)methyl)-1H-benzo[d]-imidazole-7-carboxylate (Ie) - using aqueous hydroxylamine

Following the procedure described in Example 16 using the nitrile (**IVe**), 89.2 % of the product were obtained containing, according to HPLC, 84.4 % of the product (**Ie**), 2.1 % of the starting nitrile (**IVe**) and 5.2 % of the desethyl nitrile (**VIe**). By crystallizing a sample from isopropyl acetate, a product (according to HPLC) was obtained, having a melting point of 222-238 °C (dec.), containing 92.4 % of the product (**Ie**), and 7.6 % of the desethyl nitrile (**VIe**) according to HPLC.

### Example 21

### Methyl 2-ethoxy-1-((2'-((hydroxyamino)iminomethyl)biphenyl-4-yl)methyl)-1H-benzo[d]-imidazole-7-carboxylate (Ia) - using aqueous hydroxylamine

A 50 % (w/w) aqueous solution of hydroxylamine (5 ml) was added to a stirred mixture of the nitrile (**IVa**, 4.1 g, 10 mmol) and DMSO (30 ml) and the mixture was stirred in a closed pressure flask at the temperature of 75 °C for 18 h. According to HPLC, the mixture contains 78.2 % of the product (**Ia**), 0.2 % of the starting nitrile (**IVa**) and 2.2 % of the desethyl nitrile (**VIa**). After cooling, the mixture was poured into water (50 ml), and then the insoluble fraction was aspirated and washed with water (50 ml). After drying, 4.1 g of the product (92.2 %) were obtained containing, according to HPLC, 99.1 % of the product (**Ia**), an undetectable amount of the starting nitrile (**IVa**) and 0.1 % of the desethyl nitrile (**VIa**). Crystallization from isopropyl acetate afforded a product with the melting point of 206-209 °C, containing, according to HPLC, 99.9 % of the product (**Ia**).

### Example 22

### Ethyl 2-ethoxy-1-((2'-((hydroxyamino)iminomethyl)biphenyl-4-yl)methyl)-1H-benzo[d]-imidazole-7-carboxylate (Ib) - using aqueous hydroxylamine

A 50 % (w/w) aqueous solution of hydroxylamine (5 ml) was added to a stirred mixture of the nitrile (**IVb**, 8.6 g, 20 mmol) and NMP (50 ml) and the mixture was stirred under nitrogen at the temperature of 90°C for 10 h. Then, another 50% aqueous hydroxylamine (3 ml) was added and heating was continued for another 5 h. According to HPLC, the mixture contains 83.6 % of the product (**Ib**), no detectable amount of the starting nitrile (**IVb**) and 2.2 % of the desethyl nitrile (**VIb**). After cooling, the mixture was poured into water (100 ml), and then the insoluble fraction was aspirated and washed with water (100 ml). After drying, 8.9 g of the product (97 %) were obtained, containing, according to HPLC, 96.6 % of the product (**Ib**). Crystallization from ethyl acetate afforded a product with the melting point of 209-211 °C, containing, according to HPLC, 99.7 % of the product (**Ib**).

### Example 23

### Methyl 2-ethoxy-1-((2'-((hydroxyamino)iminomethyl)biphenyl-4-yl)methyl)-1H-benzo[d] imidazole-7-carboxylate (Ia) - using aqueous hydroxylamine

A 50 % (w/w) aqueous solution of hydroxylamine (6 ml) was added to a stirred mixture of the nitrile (**IVa**, 8.2 g, 20 mmol) and DMPU (50 ml) and the mixture was stirred in a closed pressure flask at the temperature of 75 °C for 24 h. According to HPLC, the mixture contains 89.8 % of the product (**Ia**), 2.2 % of the starting nitrile (**IVa**) and 1.9 % of the desethyl nitrile (**VIa**). After cooling the mixture was poured into water (100 ml), and then the insoluble fraction was aspirated and washed with water (100 ml). After drying, 8.35 g of a product (93.9 %) were obtained, containing, according to HPLC, 99.4 % of the product (**Ia**), 0.2 % of the starting nitrile (**IVa**) and 0.2 % of the desethyl nitrile (**VIb**).

### Example 24

### Methyl 2-ethoxy-1-((2'-((hydroxyamino)iminomethyl)biphenyl-4-yl)methyl)-1H-benzo[d]-imidazole-7-carboxylate (Ia) - using aqueous hydroxylamine

A 50 % (w/w) aqueous solution of hydroxylamine (10 ml) was added to a stirred mixture of the nitrile (**IVa**, 8.2 g, 20 mmol) and NMP (50 ml) and the mixture was stirred in a closed pressure flask at the temperature of 75 °C for 48 h. According to HPLC, the mixture contains 74.0% of the product (**Ia**), no detectable amount of the starting nitrile (**IVa**) and 1.5 % of the desethyl nitrile (**VIa**). After cooling, the mixture was poured into water (100 ml), and then the insoluble fraction was aspirated and washed with water (100 ml). After drying, 7.1 of a product (79.9 %) were obtained, containing, according to HPLC, 89.4 % of the product (**Ia**), no detectable amount of the starting nitrile (**IVa**) and 2.2 % of the desethyl nitrile (**VIa**). Crystallization from tetrahydrofuran afforded a product containing, according to HPLC, 98.3 % of the product (**Ia**).

### Example 25

### Methyl 2-ethoxy-1-((2'-((hydroxyamino)iminomethyl)biphenyl-4-yl)methyl)-1H-benzo[d]-imidazole-7-carboxylate (Ia) - using aqueous hydroxylamine

A 50 % (w/w) aqueous solution of hydroxylamine (5 ml) was added to a stirred mixture of the nitrile (**IVa**, 4.1 g, 10 mmol), NMP (20 ml) and toluene (5 ml) and the mixture was stirred in a closed pressure flask at the temperature of 90 °C for 18 h. According to HPLC, the mixture contains 76.3 % of the product (**Ib**), no detectable amount of the starting nitrile (**IVb**) and 1.8 % of the desethyl nitrile (**VIb**). After cooling, the mixture was poured into water (50 ml), and then the insoluble fraction was aspirated and washed with water (50 ml). After drying, 3.7 g of a product (83.2 %) were obtained, containing, according to HPLC, 88.5 % of the product (Ib), no detectable amount of the starting nitrile (**IVb**) and 2.7 % of the desethyl nitrile (**VIb**). Crystallization from 1,2-dimethoxyethane afforded a product, containing, according to HPLC, 99.3 % of the product (**Ia**).

### Example 26

### 2-Ethoxy-1-((2'-((hydroxyamino)iminomethyl)biphenyl-4-yl)methyl)-1H-benzo[d]imidazole-7-carboxylic acid (If) - using aqueous hydroxylamine

A 50 % (w/w) aqueous solution of hydroxylamine (0.3 ml) was added to a stirred mixture of the acid (**IVf**, 0.4 g, 1 mmol) and DMSO (3 ml) and the mixture was stirred in a vial at the temperature of 50 °C for 24 h. After cooling, the mixture was poured into water (10 ml) and after acidification with acetic acid, the separated insoluble fraction was aspirated and washed with water. After drying, 0.4 g of a product (91.6 %) was obtained, containing, according to HPLC, 86.5 % of the product (**If**). Repeated crystallization from ethyl acetate afforded a product containing, according to HPLC, 98.7 % of the product (**If**) with the melting point of 208-212 °C. ¹H NMR spectrum (DMSO-*d6*): 13.19 (bs, 1H, COOH), 9.22 (bs, 1H, OH), 7.65 (dd, *J* = 7.9, 1.2 Hz, 1H, Ar), 7.53 (dd, *J* = 7.9, 1.2 Hz, 1H, Ar), 7.45-7.32 (m, 3H, Ar), 7.35 (d, *J* = 8.4 Hz, 2H, Ar), 7.29 (dd, *J* = 7,6, 1.5 Hz, 1H, Ar), 7.17 (t, *J* = 7.9 Hz, 1H, Ar), 6.99 (d, *J* = 8.4 Hz, 2H, Ar), 5.65 (s, 2H, N-CH₂-Ar), 5.57 (bs, 2H, NH₂), 4.61 (q, *J* = 7.1 Hz, 2H, OCH₂CH₃), 1.42 (t, *J* = 7.1 Hz, 3H, OCH₂CH₃).

## Claims

1. A method of manufacturing 2-ethoxy-1-((2'-((hydroxyamino)iminomethyl)biphenyl-4-yl)methyl)-1*H*-benzo[*d*]imidazole-7-carboxylic acid and its esters of formula I, 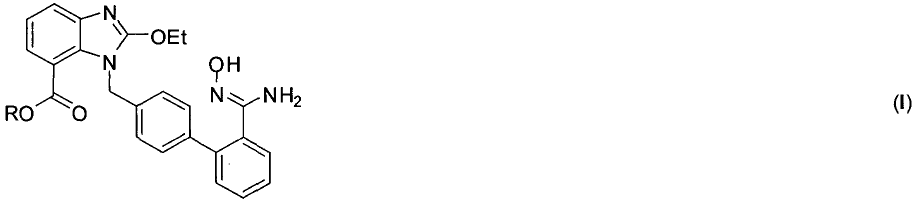 wherein R is either H or an (un)branched C₁-C₄ alkyl, ArCH₂, Ar₂CH, or Ar₃C, wherein Ar is a (un)substituted phenyl,
**characterized in that** the corresponding nitrile of formula **IV** 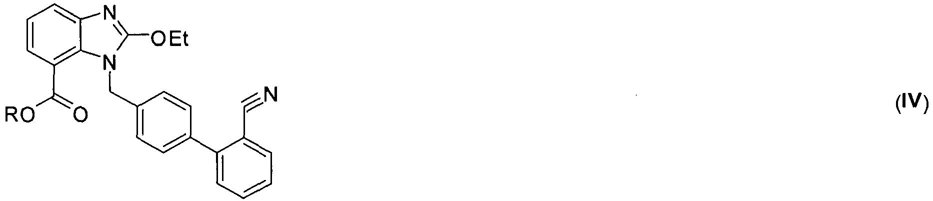 wherein R is defined as above, is reacted with an aqueous solution containing 20 to 80 weight % of hydroxylamine in a polar aprotic solvent, or in a mixture of polar aprotic solvents.

2. The method according to claim 1, **characterized in that** R is H, methyl, ethyl, benzyl, benzhydryl, or trityl.

3. The method according to claim 1, **characterized in that** the reaction is carried out with an aqueous solution of hydroxylamine containing 40 to 60 weight % of hydroxylamine.

4. The method according to any one of claims 1 to 3, **characterized in that** the reaction is carried out at a temperature of from 40 to 100 °C.

5. The method according to claim 1, **characterized in that** the polar aprotic solvent is selected from the group consisting of dimethyl sulfoxide, *N,N-*dimethyl formamide, *N,N-*dimethyl acetamide, 1-methylpyrrolidone, 1,1,3,3-tetramethylurea, 1,3-dimethylimidazolidin-2-one, 1,3-dimethyl-3,4,5,6-tetrahydro-2(1*H*)-pyrimidinone, and hexamethylphosphoramide, and mixtures thereof.

6. The method according to claim 5, **characterized in that** said solvent is DMSO or NMP.

7. The method according to any one of claims 1 to 6, **characterized in that** the reaction mixture is diluted with water, or the reaction mixture is poured into water and subsequently the insoluble product is isolated by filtration or centrifugation.

8. A method of purification of the compounds of formula **I characterized in** being obtained by the method of any one of claims 1 to 7, further **characterized in** crystallization from suitable solvents selected from the group including esters of aliphatic acids, e.g., methyl acetate, ethyl acetate, isopropyl acetate, amides, e.g., *N*,*N-*dimethyl formamide, *N,N-*dimethyl acetamide, 1-methylpyrrolidone, ethers, e.g. tetrahydrofuran, dioxan, 1,2-dimethoxyethane, 1-methoxy-2-(2-methoxyethoxy)ethane, and mixtures thereof.

## Patentansprüche

1. Verfahren zur Herstellung von 2-Ethoxy-1-((2'-((hydroxy-amino)iminomethyl)biphenyl-4-yl)methyl)-1*H*-benzo[*d*]imidazol-7-carbonsäure, und deren Ester, der Formel I 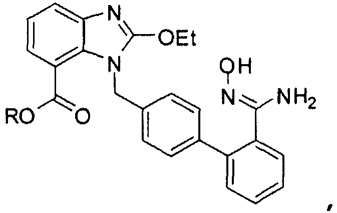 in welcher R entweder H oder ein (un)verzweigtes C₁-C₄-Alkyl, ArCH₂, Ar₂CH oder Ar₃C ist,
wobei Ar ein (un)substituiertes Phenyl ist,
**dadurch gekennzeichnet, dass** das entsprechende Nitril der Formel IV 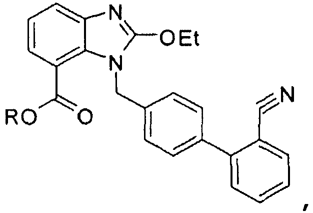 in welcher R wie oben definiert ist, mit einer 20 bis 80 Gew.% Hydroxylamin enthaltenden wässrigen Lösung in einem polaren aprotischen Lösungsmittel oder in einem Gemisch aus polaren aprotischen Lösungsmitteln umgesetzt wird.

2. Verfahren nach Anspruch 1, **dadurch gekennzeichnet, dass** R H, Methyl, Ethyl, Benzyl, Benzhydryl oder Trityl ist.

3. Verfahren nach Anspruch 1, **dadurch gekennzeichnet, dass** die Reaktion mit einer wässrigen Lösung von Hydroxylamin durchgeführt wird, die 40 bis 60 Gew% Hydroxylamin enthält.

4. Verfahren nach einem der Ansprüche 1 bis 3, **dadurch gekennzeichnet, dass** die Reaktion bei einer Temperatur von 40 bis 100 °C durchgeführt wird.

5. Verfahren nach Anspruch 1, **dadurch gekennzeichnet, dass** das polare aprotische Lösungsmittel aus der Gruppe ausgewählt ist, die aus Dimethylsulfoxid, N,N-Dimethylformamid, *N*,*N*-Dimethylacetamid, 1-Methylpyrrolidon, 1,1,3,3-Tetramethylharnstoff, 1,3-Dimethylimidazolidin-2-on, 1,3-Dimethyl-3,4,5,6-tetrahydro-2(1*H*)-pyrimidinon und Hexamethylphosphorsäuretriamid, und Gemischen davon, besteht.

6. Verfahren nach Anspruch 5, **dadurch gekennzeichnet, dass** das Lösungsmittel DMSO oder NMP ist.

7. Verfahren nach einem der Ansprüche 1 bis 6, **dadurch gekennzeichnet, dass** das Reaktionsgemisch mit Wasser verdünnt wird oder das Reaktionsgemisch in Wasser gegossen wird, und anschließend das unlösliche Produkt durch Filtration oder Zentrifugieren isoliert wird.

8. Ein Verfahren zur Reinigung der Verbindungen der Formel I, **dadurch gekennzeichnet, dass** die Verbindungen durch das Verfahren nach einem der Ansprüche 1 bis 7 erhalten wurden, weiterhin **gekennzeichnet durch** Kristallisation aus geeigneten Lösungsmitteln die aus der Ester von aliphatischen Säuren, beispielsweise Methylacetat, Ethylacetat, Isopropylacetat, Amide, beispielsweise *N*,*N*-Dimethylformamid, N,N-Dimethylacetamid, 1-Methylpyrrolidon, Ether, beispielsweise Tetra-hydrofuran, Dioxan, 1,2-Dimethoxyethan, 1-Methoxy-2-(2-methoxyethoxy)ethan, und Mischungen davon, umfassenden Gruppe ausgewählt sind.

## Revendications

1. Procédé de fabrication de l'acide 2-éthoxy-1-{[2'-(hydroxyamino-iminométhyl)-biphényl-4-yl]-méthyl}-1H-benzo-[d]-imidazole-7-carboxylique de formule I et ses esters, 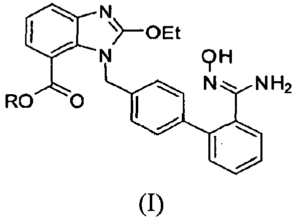 dans laquelle formule R est soit un atome d'hydrogène soit un groupe (C₁-C₄)-alkyle ramifié ou non ramifié, ArCH₂, Ar₂CH ou Ar₃C, où Ar est un groupe phényle substitué ou non substitué,
**caractérisé en ce que** le nitrile correspondant de formule IV 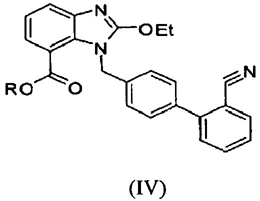 dans laquelle R est défini comme ci-dessus, est mis à réagir avec une solution aqueuse contenant 20 à 80% en poids d'hydroxylamine dans un solvant aprotique polaire ou dans un mélange de solvants aprotiques polaires.

2. Le procédé selon la revendication 1, **caractérisé en ce que** R représente un atome d'hydrogène, un groupe méthyle, éthyle, benzyle, benzhydryle ou trityle.

3. Le procédé selon la revendication 1, **caractérisé en ce que** la réaction est effectuée avec une solution aqueuse d'hydroxylamine contenant de 40 à 60% en poids d'hydroxylamine.

4. Le procédé selon l'une quelconque des revendications 1 à 3, **caractérisé en ce que** la réaction est effectuée à une température comprise entre 40 et 100° C.

5. Le procédé selon la revendication 1, **caractérisé en ce que** le solvant aprotique polaire est choisi dans le groupe constitué par le diméthylsulfoxyde, le N,N-diméthylformamide, le N, N-diméthylacétamide, la 1-méthylpyrrolidone, la 1,1,3,3-tétraméthylurée, le 1, 3 diméthylimidazolidin-2-one, le 1, 3-diméthyl-3,4,5,6-tétrahydro-2 (IH) -pyrimidinone et l'hexaméthylphosphoramide et des mélanges de ceux-ci.

6. Le procédé selon la revendication 5, **caractérisé en ce que** ledit solvant est le DMSO ou la NMP.

7. Le procédé selon l'une quelconque des revendications 1 à 6, **caractérisé en ce que** le mélange réactionnel est dilué avec de l'eau, ou le mélange réactionnel est versé dans de l'eau, et ultérieurement le produit insoluble est isolé par filtration ou centrifugation.

8. Un procédé de purification des composés de formule I **caractérisés en ce qu'**ils ont été obtenus par le procédé selon l'une quelconque des revendications 1 à 7, **caractérisé en outre en ce que** la cristallisation est réalisée dans des solvants appropriés choisis dans le groupe comprenant les esters d'acides aliphatiques, par exemple l'acétate de méthyle, l'acétate d'éthyle, l'acétate d'isopropyle, des amides, par exemple le N,N-diméthylformamide, le N,N-diméthyl-acétamide, la 1-méthylpyrrolidone, les éthers, par exemple, le tétrahydrofuranne, le dioxanne, le 1,2-di-méthoxyéthane, le 1-méthoxy-2-(2-méthoxyéthoxy)-éthane, et des mélanges de ceux-ci.
